# EUROPEAN PATENT APPLICATION

(11) **EP 4 772 636 A1**
(43) Date of publication of application: **08.07.2026**
(21) Application number: 24858750.3
(22) Date of filing: 30.08.2024
(51) Int. Cl.: C12N 15/113, C12N 15/85, C12N 15/10, A61K 35/17, A61P 35/00

(54) **RECOMBINANT PROMOTER SYSTEM SPECIFICALLY INDUCING GENE EXPRESSION AFTER T CELL ACTIVATION**

(30) Priority: 30.08.2023 CN 202311106811
(71) Applicant: Xuzhou Medical University, Xuzhou, Jiangsu 221004 (CN)
(72) Inventor: DU, Hongwei, Xuzhou, Jiangsu 221004 (CN); LI, Fei, Xuzhou, Jiangsu 221004 (CN); LI, Zhudi, Xuzhou, Jiangsu 221004 (CN); WANG, Chen, Xuzhou, Jiangsu 221004 (CN); ZHENG, Junnian, Xuzhou, Jiangsu 221004 (CN)
(74) Representative: Valet Patent Services Limited
(86) International application number: PCT/CN2024/115991
(87) International publication number: WO 2025/045221

(57) **Abstract**

Provided is a recombinant promoter system specifically inducing gene expression after T cell activation. Also provided is a new T cell activation-induced recombinant promoter constructed by modifying and optimizing GM-CSF, IFNg and IL2 promoters. When CAR-T cells are activated, the recombinant promoter can efficiently induce the expression of downstream genes, with the activity thereof being superior to that of a traditional NFAT-responsive promoter constructed based on a binding sequence of a nuclear factor of activated T cells. Further provided are a CAR-T cell comprising the T cell activation-induced recombinant promoter and the use thereof.

## Description

### TECHNICAL FIELD

The present application relates to the field of biological medicines. Specifically, the present application relates to a recombinant promoter system specifically inducing gene expression after T cell activation.

### BACKGROUND ART

Chimeric antigen receptor T-cell (CAR-T) immunotherapy is an adoptive T-cell therapy that uses a genetic engineering technique to enable T cells to express receptors capable of specifically recognizing antigens and co-stimulatory signaling molecules, then expanding the cells in vitro and reinfusing into the body of the patient to enable them to recognize tumor-associated antigens and kill tumor cells independently of major histocompatibility complex (MHC) presentation. In recent years, CAR-T cell therapy has achieved significant success in the treatment of hematological malignancies. Several CAR-T therapies have been approved currently for treating hematological malignancies. Among them, CD19-targeted CAR-T cell therapy can achieve a remission rate of 90% or above in refractory and relapsed acute lymphoblastic leukemia; and BCMA-targeted CAR-T cell therapy has an overall response rate (ORR) of up to 96% in the treatment of multiple myeloma, offering hope for curing tumor with CAR-T therapy.

While CAR-T therapy has achieved great success in the clinical treatment of hematological malignancies, its clinical efficacy in solid tumors remains very limited, still facing numerous difficulties and challenges: the antigen heterogeneity problem is prevalent in solid tumors, leading to antigen escape; CAR-T cells cannot effectively infiltrate solid tumor tissues; CAR-T cells have poor persistence and are easily depleted; the immunosuppressive microenvironment in solid tumors inhibits CAR-T function; and off-target effects result in severe toxic side effects. Therefore, many strategies have been attempted to modify and optimize CAR-T to enable them to have lower toxicity while exhibiting optimal activity, thereby enhancing their efficacy in solid tumors.

Besides optimizing and modifying the chimeric antigen receptor itself, a relatively effective strategy is to engineer CAR-T cells to express relevant functional proteins, to enhance their killing capabilities by "arming" the CAR-T cells. For example, they are engineered to express immunomodulatory factors (such as cytokines, immune checkpoint inhibitory molecules, and bacterial virulence factor proteins) to effectively improve CAR-T persistence and activate the immune system. Engineered T cells overexpressing γ-chain cytokines (such as IL2, IL7, IL15, and IL21) or inflammatory cytokines (such as IL12, IL18, and IL23) show better antitumor activity. However, persistent expression of immunomodulatory factors may cause severe toxic side effects, thereby preventing its advantages from being fully realized. For example, CAR-T cells overexpressing IL2 or IL18 have been found to cause severe toxic side effects in preclinical studies, limiting their application in clinical translation. Therefore, strictly controlling the expression of these loaded proteins (transgenic proteins) and ensuring their secretion only within the tumor lesion can maximize the functional advantages of the loaded proteins and simultaneously reduce their influence on normal tissues.

Therefore, there is a need in the art to develop a method that enables T cells to express immunomodulatory factors highly efficiently and highly specifically.

### SUMMARY

The objective of the present application is to provide a recombinant promoter expression system specifically inducing gene expression after T cell activation.

Another objective of the present application is to provide a T cell containing the recombinant promoter expression system of the present application and the use thereof.

In a first aspect of the present application, a genetically modified promoter element is provided, the promoter element is a T cell activation induced promoter, and the promoter element has a fragment derived from a promoter selected from the group consisting of: GM-CSF promoter, IFNg promoter, IL2 promoter, and combinations thereof.

In another preferred embodiment, the T cell activation induced promoter refers to a promoter that regulates a target gene that is not expressed or expressed at a low level when T cells are not activated and highly expressed when T cells are activated.

In another preferred embodiment, the promoter element performs induction by a T-cell activation pathway selected from the group consisting of:
a) induction by binding to a ligand of a T-cell receptor (TCR);
b) induction by binding to a ligand of a chimeric antigen receptor (CAR);
c) activation by antibodies against immune cell surface markers (including anti-CD3 and anti-CD28); and
d) activation by a compound activator (such as PMA, inomycin).

In another preferred embodiment, under a T-cell activation condition, the transcriptional efficiency of the promoter element is ≥50%, preferably ≥100%, and more preferably ≥150% higher than that of the nuclear factor of activated T cells (NFAT) promoter.

In another preferred embodiment, the promoter element has the structure as shown in following formula (I):

Z0-Z1-Z2 (I)

in the formula, "-" represents a bond or nucleotide linker sequence;
Z0 is none, a fragment derived from a minimal promoter, or a fragment derived from other promoters;
Z1 is a fragment derived from a promoter selected from the group consisting of: GM-CSF promoter, IFNg promoter, IL2 promoter, and combinations thereof; and
Z2 is none, a fragment derived from the minimal promoter, or a fragment derived from other promoters.

In another preferred embodiment, Z0 is none; and Z2 is the fragment derived from the minimal promoter.

In another preferred embodiment, Z2 is none; and Z0 is the fragment derived from the minimal promoter.

In another preferred embodiment, Z1 is a GM-CSF promoter fragment.

In another preferred embodiment, the nucleotide sequence length of the fragment is 50-800 bp, preferably 150-700 bp, and more preferably 200-400 bp.

In another preferred embodiment, Z1 is a nucleic acid region from position p1 to q1 derived from a human GM-CSF promoter, where
p1 is any integer from -800 to -100, and preferably from -400 to -150; and
q1 is any integer from -50 to 50, preferably from -40 to +40, and more preferably from -36 to +36.

In another preferred embodiment, Z1 is a nucleic acid region from position -626 (preferably -336) to +36 (preferably +35) of the human GM-CSF promoter, or a truncated fragment thereof.

In another preferred embodiment, Z1 includes a nucleic acid region from position -217 to -1 of the GM-CSF promoter.

In another preferred embodiment, the promoter element includes:
(a) a nucleotide sequence as shown in any one of SEQ ID NOs: 11-17;
(b) a nucleotide sequence having ≥90% (preferably ≥95%, more preferably ≥98%) homology to the sequence as shown in any one of SEQ ID NOs: 11-17, and having or substantially having the function of polynucleotide as shown in any one of SEQ ID NOs: 11-17; or
(c) a polynucleotide that has one or more, preferably 1-30, and more preferably 1-6 nucleotides, truncated at the 5' end and/or 3' end of the polynucleotide as shown in any one of SEQ ID NOs: 11-17, and has or substantially has the function of the polynucleotide as shown in any one of SEQ ID NOs: 11-17.

In another preferred embodiment, Z1 is an IL-2 promoter fragment.

In another preferred embodiment, the nucleotide sequence length of the fragment is 200-600 bp, preferably 300-500 bp, and more preferably 350-400 bp.

In another preferred embodiment, Z1 is the nucleic acid region from position p2 to q2 derived from a human IL-2 promoter, where
p2 is any integer from -500 to -200, and preferably from -400 to -300; and
q2 is any integer from 0 to +100, preferably from +30 to +70, and more preferably from +40 to +60.

In another preferred embodiment, Z1 includes the nucleic acid region from position - 326 to +47 of the IL-2 promoter.

In another preferred embodiment, the promoter element includes:
(a) a nucleotide sequence as shown in SEQ ID NO: 18;
(b) a nucleotide sequence having ≥90% (preferably ≥95%, more preferably ≥98%) homology to the sequence as shown in SEQ ID NO: 18 and substantially having the function of the polynucleotide as shown in SEQ ID NO: 18; or
(c) a polynucleotide that has one or more, preferably 1-30, more preferably 1-6 nucleotides, truncated at the 5' end and/or 3' end of the polynucleotide as shown in SEQ ID NO: 18, and substantially has the function of the polynucleotide as shown in SEQ ID NO: 18.

In another preferred embodiment, Z1 is an IFNg promoter segment.

In another preferred embodiment, the nucleotide sequence length of the fragment is 100-1000 bp, preferably 300-800 bp, and more preferably 500-700 bp.

In another preferred embodiment, Z1 is the nucleic acid region from position p3 to q3 derived from the IFNg promoter, where
p3 is any integer from -800 to -100, and preferably from -600 to -400; and
q3 is any integer from +20 to +120, preferably from +40 to +80, and more preferably from +50 to +70.

In another preferred embodiment, Z1 is the nucleic acid region from position -538 to +64 of the IFNg promoter, or a truncated fragment thereof.

In another preferred embodiment, Z1 includes the nucleic acid region from position - 108 to +64 of the IFNg promoter.

In another preferred embodiment, the promoter element includes:
(a) a nucleotide sequence as shown in SEQ ID NO: 19 or 20;
(b) a nucleotide sequence having ≥90% (preferably ≥95%, more preferably ≥98%) homology to the sequence as shown in SEQ ID NO: 19 or 20, and substantially having the function of the polynucleotide as shown in SEQ ID NO: 19 or 20; or
(c) a polynucleotide that has one or more, preferably 1-30, more preferably 1-6 nucleotides, truncated at the 5' end and/or 3' end of the polynucleotide as shown in SEQ ID NO: 19 or 20, and substantially has the function of the polynucleotide as shown in SEQ ID NO: 19 or 20.

In another preferred embodiment, the minimal promoter is a constitutive promoter or an inducible promoter.

In another preferred embodiment, the minimal promoter is selected from the group consisting of: minimal CMV promoter, minimal IL2 promoter, synthetic minimal promoter, and any other minimal promoter.

In another preferred embodiment, the nucleotide sequence of the fragment derived from the minimal promoter is as shown in SEQ ID NO: 21.

In another preferred embodiment, the nucleotide sequence of the promoter element is:
(a) a nucleotide sequence as shown in any one of SEQ ID NOs: 22-29;
(b) a nucleotide sequence having ≥90 % (preferably ≥95%, more preferably ≥98%) homology to the sequence as shown in any one of SEQ ID NOs: 22-29, and substantially having the function of the polynucleotide as shown in any one of SEQ ID NOs: 22-29; or
(c) a polynucleotide that has one or more, preferably 1-30, more preferably 1-6 nucleotides, truncated at the 5' end and/or 3' end of the polynucleotide as shown in any one of SEQ ID NOs: 22-29, and substantially has the function of the polynucleotide as shown in any one of SEQ ID NOs: 22-29.

In another preferred embodiment, the nucleotide sequence of the promoter element is as shown in any one of SEQ ID NOs: 11-20, and 22-29.

In a second aspect of the present application, an expression cassette is provided, the expression cassette having the following elements: the promoter element in the first aspect of the present application, and a target gene ORF sequence operatively linked to the promoter element.

In another preferred embodiment, the target gene encodes an immunomodulatory factor protein.

In another preferred embodiment, the immunomodulatory factor is selected from the group consisting of: cytokines, chemokines, antibodies, chimeric antigen receptors, and combinations thereof.

In another preferred embodiment, the cytokines include, but are not limited to, cytokines selected from the group consisting of: IL-2, IL-15, IL-12, IL-18, IL-21, IL-7, IL9, IL23, INF-α, IFN-β, IFN-γ, and combinations thereof.

In another preferred embodiment, the chemokines include, but are not limited to, cytokines selected from the group consisting of: CCL20, CCL1, CCL17, CCL22, CCL27, CCL28, CXCL9, CXLC10, and combinations thereof.

In another preferred embodiment, the antibodies and/or chimeric antigen receptors may target antigens selected from the group consisting of: PD1, 4-1BB, OX40, CD40, CTLA-4, CSF1R, FAP, and combinations thereof, but not limited thereto.

In another preferred embodiment, the immunomodulatory factor is selected from neutrophil-activating protein (NAP) and MMPs (matrix metalloproteinases).

In another preferred embodiment, the immunomodulatory factor is mouse IL2 or human IL2.

In another preferred embodiment, the expression cassette further includes one or more elements selected from the group consisting of: poly(A) elements, enhancers, transport elements, and gene-targeting elements.

In a third aspect of the present application, an expression cassette for co-expressing a chimeric antigen receptor and an immunomodulatory factor is provided, where the expression cassette has the promoter element in the first aspect of the present application, the promoter element regulating the expression of the immunomodulatory factor.

In another preferred embodiment, the chimeric antigen receptor and immunomodulatory factor are expressed in the same direction or expressed in opposite directions.

In another preferred embodiment, the expression cassette has the structure as shown in following formula II from the 5' end to the 3' end:

CAR-PA2-IF-Prom (II)

In the formula,
each "-" independently represents a bond or a linker nucleic acid sequence;
CAR is a coding sequence of the chimeric antigen receptor;
PA2 is a bidirectional polyA sequence;
IF is a coding sequence of an immunomodulatory factor in reverse orientation; and
Prom is a promoter element in the first aspect of the present application, in reverse orientation.

In another preferred embodiment, the bidirectional PolyA sequence is as shown in SEQ ID NO: 30.

In another preferred embodiment, the immunomodulatory factor is selected from the group consisting of: cytokines, chemokines, antibodies, chimeric antigen receptors, and combinations thereof.

In another preferred embodiment, the cytokines include, but are not limited to, cytokines selected from the group consisting of: IL-2, IL-15, IL-12, IL-18, IL-21, IL-7, IL9, IL23, INF-α, IFN-β, IFN-γ, and combinations thereof.

In another preferred embodiment, the chemokines include, but are not limited to, cytokines selected from the group consisting of: CCL20, CCL1, CCL17, CCL22, CCL27, CCL28, CXCL9, CXLC10, and combinations thereof.

In another preferred embodiment, the antibodies and/or chimeric antigen receptors may target antigens selected from the group consisting of: PD1, 4-1BB, OX40, CD40, CTLA-4, CSF1R, FAP, and combinations thereof, but not limited thereto.

In another preferred embodiment, the immunomodulatory factor is selected from NAP and MMPs.

In another preferred embodiment, the immunomodulatory factor is mouse IL2 or human IL2.

In another preferred embodiment, the amino acid sequence of the immunomodulatory factor is as shown in SEQ ID NO: 10.

In another preferred embodiment, the coding nucleotide sequence of the immunomodulatory factor is as shown in SEQ ID NO: 9.

In another preferred embodiment, the chimeric antigen receptors have the structure as shown in following formula III:

L-F-EB-H-TM-C-CD3ζ (III)

In the formula,
each "-" independently represents a bond or a linker nucleic acid sequence;
L is none or a coding sequence of a signal peptide;
F is none or a tag sequence;
EB is a coding sequence of an extracellular binding domain;
H is none or a hinge region;
TM is a transmembrane domain;
C is none or a co-stimulatory signaling molecule; and
CD3ζ is a cytoplasmic signaling sequence derived from CD3ζ.

In another preferred embodiment, L is a signal peptide of a protein selected from the group consisting of: CD8, CD28, GM-CSF, CD4, CD137, SECTM1, and combinations thereof.

In another preferred embodiment, the extracellular binding domain is selected from the group consisting of: scFv, a ligand, and a combination thereof.

In another preferred embodiment, the extracellular binding domain specifically binds to B7-H3.

In another preferred embodiment, the amino acid sequence of the extracellular binding domain is as shown in SEQ ID NO: 2.

In another preferred embodiment, the coding nucleotide sequence of the extracellular binding domain is as shown in SEQ ID NO: 1.

In another preferred embodiment, H is a hinge region of a protein selected from the group consisting of: CD8, CD28, CD137, antibody Fc, and combinations thereof.

In another preferred embodiment, H is a hinge region derived from CD8α.

In another preferred embodiment, the TM is a transmembrane region of a protein selected from the group consisting of: CD28, CD3 epsilon, CD45, CD4, CD5, CD8, CD9, CD16, CD22, CD33, CD37, CD64, CD80, CD86, CD134, CD137, CD154, HVEM, and combinations thereof.

In another preferred embodiment, the TM is a transmembrane region derived from CD8α.

In another preferred embodiment, the amino acid sequences of the hinge region and the transmembrane region are as shown in SEQ ID NO: 4.

In another preferred embodiment, the coding nucleotide sequences of the hinge region and the transmembrane region are as shown in SEQ ID NO: 3.

In another preferred embodiment, C is a co-stimulatory signaling molecule of a protein selected from the group consisting of: OX40, CD2, CD7, CD27, CD28, CD30, CD40, CD70, CD134, 4-1BB (CD137), PD1, Dap10, CDS, ICAM-1, LFA-1 (CD11a/CD18), ICOS (CD278), NKG2D, GITR, TLR2, HVEM, and combinations thereof.

In another preferred embodiment, C is a co-stimulatory signaling molecule derived from CD28.

In another preferred embodiment, the amino acid sequence of C is as shown in SEQ ID NO: 6.

In another preferred embodiment, the coding nucleotide sequence of C is as shown in SEQ ID NO: 5.

In another preferred embodiment, the amino acid sequence of the CD3ζ-derived cytoplasmic signaling sequence is as shown in SEQ ID NO: 8.

In another preferred embodiment, the coding nucleotide sequence of the CD3ζ-derived cytoplasmic signaling sequence is as shown in SEQ ID NO: 7.

In a fourth aspect of the present application, a vector is provided, the vector containing the expression cassette in the second aspect of the present application, or the expression cassette for co-expressing a CAR and an immunomodulatory factor in the third aspect of the present application.

In another preferred embodiment, the vector is selected from the group consisting of: DNA, RNA, plasmid, lentiviral vector, adenovirus vector, retroviral vector, transposon, and combinations thereof.

In another preferred embodiment, the vector is the retroviral vector.

In a fifth aspect of the present application, a host cell is provided, where the host cell contains the vector in the fourth aspect of the present application, or a chromosome thereof is integrated with an exogenous promoter element in the first aspect of the present application, or the chromosome thereof is integrated with the expression cassette in the second aspect of the present application.

In another preferred embodiment, the chromosome of the host cell has one or more (e.g., 1-50, preferably 2-6) copies of the promoter element in the first aspect of the present application, or the expression cassette in the second aspect of the present application.

In a sixth aspect of the present application, an engineered immune cell is provided, where the immune cell contains the vector in the fourth aspect of the present application, or a chromosome thereof is integrated with the expression cassette in the second aspect of the present application or the expression cassette for co-expressing a CAR and an immunomodulatory factor in the third aspect of the present application.

In another preferred embodiment, the engineered immune cell is selected from the group consisting of: T cell, CAR-T cell, TCR-T cell, NK cell, γδ T cell, NKT cell, macrophage, dendritic cell, neutrophil, and combinations thereof.

In another preferred embodiment, the engineered immune cell is chimeric antigen receptor T cell (CAR-T cell), T cell receptor gene-modified T cell (TCR-T cell), or chimeric antigen receptor NK cell (CAR-NK cell).

In a seventh aspect of the present application, a method for preparing the engineered immune cell in the sixth aspect of the present application is provided, including the following steps of: transducing the expression cassette in the second aspect of the present application, or the expression cassette for co-expressing a CAR and an immunomodulatory factor in the third aspect of the present application, or the vector in the fourth aspect of the present application, into an immune cell, to obtain the engineered immune cell.

In an eighth aspect of the present application, a pharmaceutical composition is provided, where the pharmaceutical composition includes the vector in the fourth aspect of the present application, the host cell in the fifth aspect of the present application, and/or the engineered immune cell in the sixth aspect of the present application, and a pharmaceutically acceptable carrier, diluent, or excipient.

In another preferred embodiment, the formulation is a liquid formulation.

In another preferred embodiment, the dosage form of the formulation is an injection.

In another preferred embodiment, the concentration of the engineered immune cell in the formulation is 1×10³-1×10⁸ cells/ml, and preferably 1×10⁴-1×10⁷ cells/ml.

In a ninth aspect of the present application, a use of the vector in the fourth aspect of the present application, the host cell in the fifth aspect of the present application, or the engineered immune cell in the sixth aspect of the present application is provided, for preparation of a medicament or a formulation for preventing and/or treating a disease.

In another preferred embodiment, the disease is a tumor.

In another preferred embodiment, the tumor includes solid tumor and hematologic malignancy.

In another preferred embodiment, the solid tumor is selected from the group consisting of: breast cancer, lung cancer, pancreatic cancer, osteosarcoma, gastric cancer, colorectal cancer, hepatobiliary cancer, bladder cancer, non-small cell lung cancer, ovarian cancer, cervical cancer, esophageal cancer, glioma, prostate cancer, nasopharyngeal carcinoma, head and neck cancer, and biliary tract cancer.

In another preferred embodiment, the hematologic malignancy is selected from the group consisting of: acute leukemia, chronic leukemia, B-cell lymphoma, T-cell lymphoma, non-Hodgkin's lymphoma, and multiple myeloma.

In another preferred embodiment, the disease is a disease with high expression of B7-H3.

In a tenth aspect of the present application, a method for treating a disease is provided, including administering to a subject in need of treatment an effective amount of the host cell in the fifth aspect of the present application, or the engineered immune cell in the sixth aspect of the present application, or the pharmaceutical composition in the eighth aspect of the present application.

In another preferred embodiment, the disease is a tumor.

In another preferred embodiment, the tumor includes solid tumor and hematologic malignancy.

In another preferred embodiment, the solid tumor is selected from the group consisting of: breast cancer, lung cancer, pancreatic cancer, osteosarcoma, gastric cancer, colorectal cancer, hepatobiliary cancer, bladder cancer, non-small cell lung cancer, ovarian cancer, cervical cancer, esophageal cancer, glioma, prostate cancer, nasopharyngeal carcinoma, head and neck cancer, and biliary tract cancer.

In another preferred embodiment, the hematologic malignancy is selected from the group consisting of: acute leukemia, chronic leukemia, B-cell lymphoma, T-cell lymphoma, non-Hodgkin's lymphoma, and multiple myeloma.

In another preferred embodiment, the disease is a disease with high expression of B7-H3.

In another preferred embodiment, the CAR immune cell contained in the engineered immune cell or pharmaceutical composition is a cell derived from the subject (autologous cell).

In another preferred embodiment, the CAR immune cell contained in the engineered immune cell or pharmaceutical composition is a cell derived from a healthy individual (allogeneic cell).

In another preferred embodiment, the method may be used in combination with other treatment methods.

In another preferred embodiment, the other treatment methods include chemotherapy, radiotherapy, targeted therapy, etc.

It should be understood that, within the scope of the present application, the above-described technical features of the present application and the technical features specifically described below (such as in the embodiments) may be combined with each other to form new or preferred technical solutions. Due to space limitations, they will not be described in detail here.

### BRIEF DESCRIPTION OF DRAWINGS

The following drawings are used to illustrate specific embodiments of the present application and are not intended to limit the scope of the present application as defined by Claims.

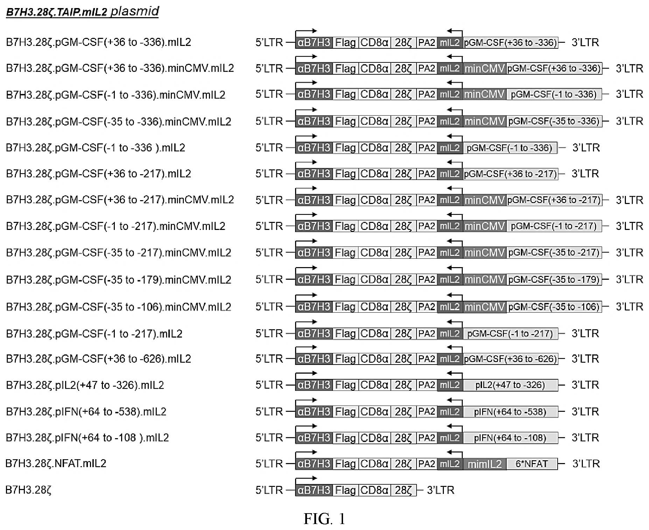

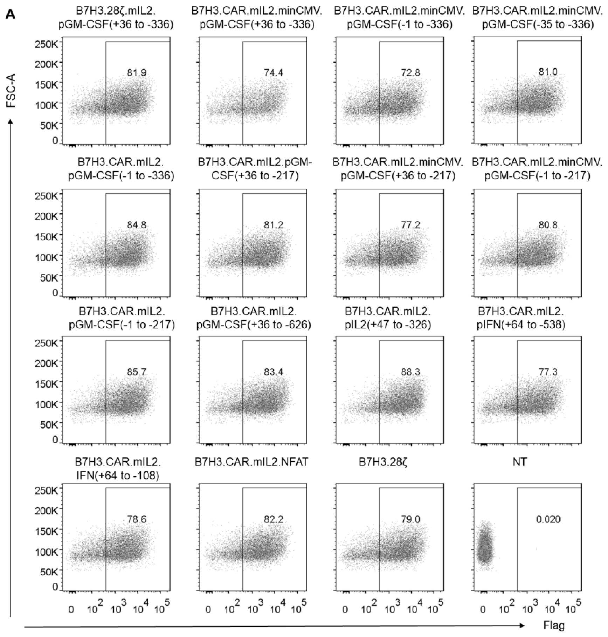

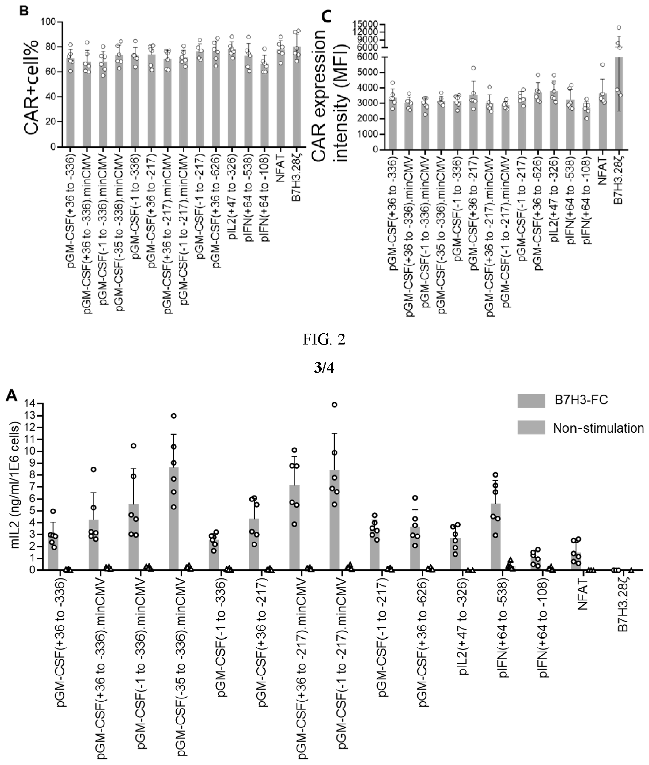

FIG. 1 is a schematic view showing molecular structures of vectors (B7H3.28ζ.TAIP.mIL2) for co-expressing the B7H3.28ζ CAR molecule and gene expression systems induced by different versions of T cell activation induced promoter (TAIP). Among them, aB7H3: anti-B7H3 antibody scFv, CD8: hinge region and transmembrane domain of CD8α, 28ζ: intracellular co-stimulatory domain of CD28 and intracellular domain of CD3ζ, Flag: Flag protein, PA2: bidirectional PolyA sequence, mIL2: mouse IL2 gene, pGM-CSF: GM-CSF promoter, pIFN: IFN promoter, pIL2: IL2 promoter, minCMV: minimal CMV promoter, 6*NFAT: hexameric repeat of the NFAT binding sequence, minIL2: minimal IL2 promoter, pGM-CSF (+36 to -217) represents the nucleic acid region from position +36 to -217 of the GM-CSF promoter, and so on.
FIG. 2 shows the expression efficiencies of different versions of the B7H3.28ζ.TAIP.mIL2 vectors in T cells. FIG. 2A is a representative flow cytometry plot of the infection rate of B7H3.28ζ.TAIP.mIL2 in T cells on day 4 after infecting the T cells, detected by using anti-Flag antibody, where NT (Non-transduced T) is the control group in which T cells are not transduced. This figure represents at least 6 independent experiments with similar results. FIG. 2B is a statistical graph of the infection rate of B7H3.28ζ.TAIP.mIL2 in T cells. FIG. 2C is a statistical graph of the expression intensity of B7H3.28ζ.TAIP.mIL2 in T cells.

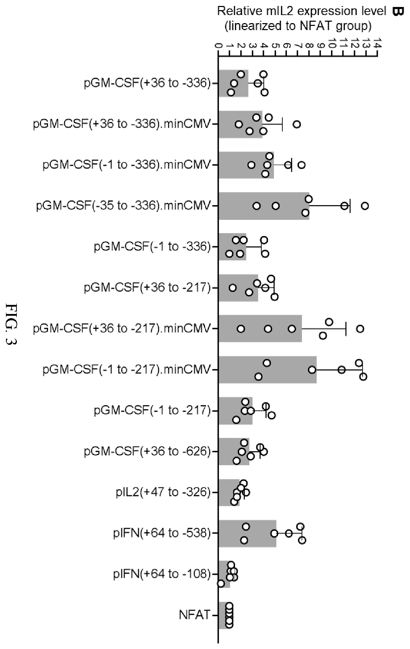

FIG. 3 shows detection results of gene expression levels induced by different versions of TAIP in response to T cell activation signals. FIG. 3A shows the secretion level of mIL2 in the supernatant detected by ELISA after T cells are infected with B7H3.28ζ.TAIP.mIL2 for 7 days and then stimulated with B7H3-FC-coated antigen for 24 h. FIG. 3B shows the relative expression levels of mIL2 induced by different TAIPs after T cell activation, where the mIL2 expression level is first calibrated with CAR expression intensity and then compared with the expression level induced by the NFAT promoter group.

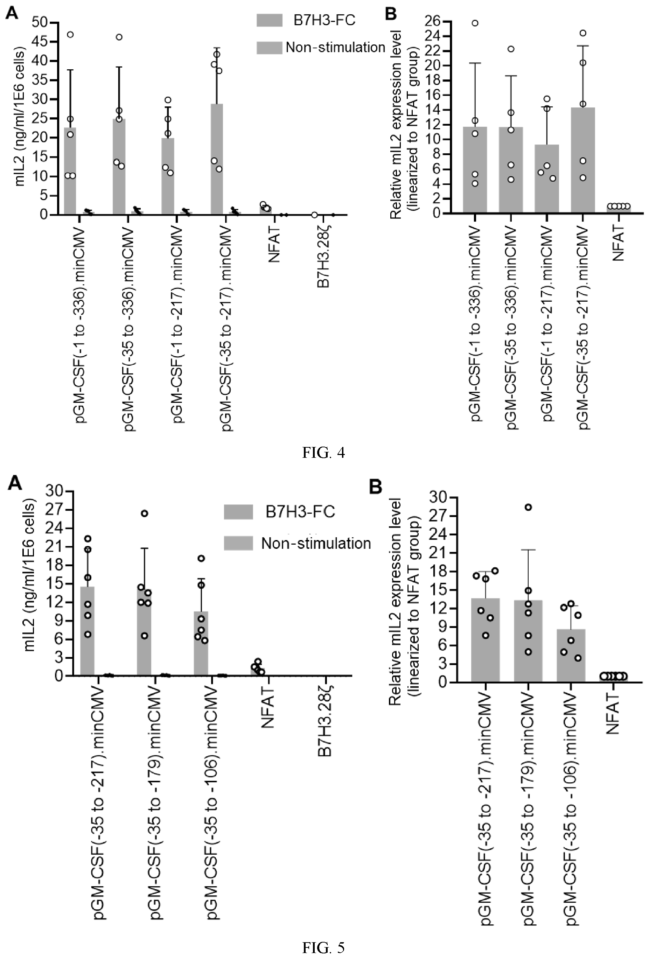

FIG. 4 shows the detection results of the induction activity of recombinant promoters including different pGM-CSF fragments and the minCMV. FIG. 4A shows the secretion level of mIL2 (detected by ELISA) induced by different pGM-CSF-minCMV recombinant promoters after 24 hours of T cell activation. FIG. 4B shows the relative expression levels of mIL2 induced by different pGM-CSF-minCMV recombinant promoters after T cell activation, where the mIL2 expression level is first calibrated with CAR expression intensity and then compared with the expression level induced by the NFAT promoter group.
FIG. 5 shows the detection results of the induction activity of recombinant promoters including different pGM-CSF fragments and the minCMV. FIG. 5A shows the secretion level of mIL2 (detected by ELISA) induced by different pGM-CSF-minCMV recombinant promoters after 24 hours of T cell activation. FIG. 5B shows the relative expression levels of mIL2 induced by different pGM-CSF-minCMV recombinant promoters after T cell activation, where the mIL2 expression level is first calibrated with CAR expression intensity and then compared with the expression level induced by the NFAT promoter group.

### DETAILED DESCRIPTION OF EMBODIMENTS

Through extensive and in-depth research, the inventors have developed, for the first time, a novel T cell activation induced recombinant promoter constructed by modifying and optimizing the GM-CSF, IFNg, and IL2 promoters. The recombinant promoter of the present application can efficiently induce the expression of downstream genes of CAR-T cell during activation thereof, and the activity thereof is superior to that of traditional NFAT-responsive promoters constructed based on the NFAT (nuclear factor of activated T cells) binding sequence. This type of T cell activation induced recombinant promoter can be used to induce CAR-T cells to secrete and express various immunomodulatory molecules such as cytokines and antibody-like proteins in tumors, to promote the activity and persistence of CAR-T cells and thereby facilitate the application of CAR-T cells in solid tumors. Based on this, the present application was completed.

### Terms

Unless otherwise defined, all technical and scientific terms used herein have the same meaning as commonly understood by one of ordinary skill in the art to which the present application pertains. As used herein, when used for a specifically enumerated numerical value, the term "about" means that the value may vary from the enumerated value by no more than 1%. For example, as used herein, the expression "about 100" includes all values between 99 and 101 (e.g., 99.1, 99.2, 99.3, 99.4, etc.).

As used herein, the terms "optional" or "optionally" mean that the events or circumstances described subsequently may occur but does not have to occur.

As used herein, the terms "containing" or "including (comprising)" may be open-ended, semi-closed, or closed. In other words, the terms also include "substantially composed of" or "composed of".

"Transduction", "transfection", "conversion" or the terms used herein refer to the process of delivering an exogenous polynucleotide to a host cell, and transcribing and translating to produce polypeptide products, including the introduction of an exogenous polynucleotide into a host cell (e.g., *Escherichia coli)* by using a plasmid molecule.

"Gene expression" or "expression" refers to the process that a gene is transcribed, translated, and modified after the translation to produce RNA or protein product(s) of the gene.

"Polynucleotide" refers to a polymerization form of nucleotides of any lengths, including deoxyribonucleotide (DNA), ribonucleotide (RNA), and hybrid sequences and analogs thereof. Polynucleotide may include modified nucleotides, such as methylated or capped nucleotides or nucleotide analogs. As used herein, the term polynucleotide refers to interchangeable single-stranded and double-stranded molecules. Unless otherwise stated, polynucleotides in any embodiment described herein include the double-stranded form and two complementary single strands that are known or predicted to constitute the double-stranded form.

Sequence identity is determined by comparing two aligned sequences along a predetermined comparison window (which may be 50%, 60%, 70%, 80%, 90%, 95%, or 100% of the length of a reference nucleotide sequence or protein) and determining the number of positions where identical residues appear. This is typically expressed as a percentage. The measurement of sequence identity of nucleotide sequences is a method well known to those skilled in the art.

The term "administering" refers to the physical introduction of the product of the present application into a subject by using any one of the various methods and delivery systems known to those skilled in the art, including intravenous, intramuscular, subcutaneous, intraperitoneal, spinal, or other parenteral routes of administration, for example, by injection or infusion.

### Promoter

As used herein, the term "promoter" refers to a nucleic acid sequence that functions to accurately and effectively initiate gene transcription. It directs the transcription of the gene's nucleic acid sequence into mRNA, and is typically present upstream (5' end) of the coding sequence of the target gene. The promoter or promoter fragment generally provides recognition site(s) for RNA polymerase and other factors necessary for correct transcription initiation.

As used herein, the term "constitutive promoter" refers to a promoter under the control of which the expression of a structural gene remains generally constant at a certain level without significant difference in the expression level in different tissues and parts.

As used herein, the term "inducible promoter" refers to a promoter that may be greatly increase the transcriptional level of a gene under the stimulation of certain specific physical or chemical signals.

As used herein, the term "T cell activation induced promoter" refers to a class of promoters that may be induced to activate and thereby initiate the expression of genes driven thereby when T cells are activated under the stimulation of certain specific physical or chemical signals.

As used herein, when applied to immune cells (such as T cells), the term "activate" or "activation" refers to the process of stimulating immune cells with corresponding stimulating factors to achieve expansion to increase the number of immune cells or activation to enhance the activity of immune cells. The stimulating factors for immune cells may be any factors capable of causing expansion or enhanced activity of immune cells. In some embodiments, the stimulating factors may also be antibodies targeting immune cell surface markers such as CD3 and CD28, or compound activators such as PMA and inomycin. In some embodiments, when immune cells express a T cell receptor (TCR) or a chimeric antigen receptor (CAR) against certain specific antigen, the stimulating factor for immune cells may also include that specific antigen or its binding epitope fragment, which induces TCR or CAR activation through contact with T cells. For example, for T cells expressing an anti-B7-H3 chimeric antigen receptor, the stimulating factor thereof may be B7-H3 expressed by tumor cells.

As used herein, the term "minimal promoter" refers to a short DNA segment which has no activity per se but can mediate strong transcription when combined with other transcriptional regulatory element. The characteristic of enhancing transcription is common to minimal promoters; therefore, the promoter element of the present application may include any minimal promoter. The minimal promoter sequence may be derived from a variety of sources, including prokaryotic and eukaryotic genes. In one embodiment, the promoter element of the present application includes the minCMV promoter, which is a minimal promoter derived from human cytomegalovirus.

The promoter of the present application has a fragment derived from the GM-CSF promoter, IFNg promoter, or IL2 promoter. The GM-CSF promoter is as set forth in Gene ID: 1437; the IFNg promoter is as set forth in Gene ID: 3458; and the IL2 promoter is as set forth in Gene ID: 3558. In the present application, the nucleotide (base) numbering of the promoter fragment is calculated according to conventional methods in the art, i.e., calculated with the transcription start site as the origin (position +1), upstream of the transcription start site recorded as a negative number and downstream of the transcription start site recorded as a positive number. For example, "the nucleic acid region from position -626 to +36 of the GM-CSF promoter" refers to the fragment of the GM-CSF promoter from position 626 upstream to position 36 downstream of the transcription start site.

In the promoter of the present application, the fragment derived from the GM-CSF promoter, IFNg promoter, or IL2 promoter and the minimal promoter (or other promoter fragment) may be directly linked or linked via an additional nucleotide sequence. In one embodiment, to facilitate the insertion of the derived promoter fragment and the minimal promoter into the vector respectively during construction, the additional nucleotide sequence used may be a restriction enzyme cutting site, for example, GCGGCCGC.

The promoter fragment used to construct the promoter element of the present application may be derived from any animal, for example, mouse, rat, rabbit, primate (e.g., human), etc.

The present application has screened out a series of T cell activation induced promoters through tests. While the present application specifically provides promoter elements as shown in SEQ ID NOs: 11-20 and 22-29, this is merely to provide exemplary embodiments to illustrate the parental promoter fragments and minimal promoters that the promoter elements of the present application may include. For example, the present application provides test results for the promoter element containing the region from position +36 to -217 of the GM-CSF promoter and promoter element containing the region from position +36 to -626 of the GM-CSF promoter. Those skilled in the art shall understand that other promoter elements between these two all can achieve the effects of the present application, for example the promoter element containing the region from position +36 to -300 (or -400, -500) of the GM-CSF promoter. Therefore, the present application includes these exemplary promoter elements and their derived promoter elements.

The recombinant promoter expression system of the present application may be used to specifically induce gene expression after T cell activation, and may be used to optimize, modify, and enhance the antitumor function of gene-edited immune cells or modify the tumor immune microenvironment, etc.

### Expression cassette

The present application provides an expression cassette having the promoter element in the first aspect of the present application and a target gene ORF sequence.

The promoter of the present application may be used to express any target gene. In one embodiment, the promoter of the present application is linked downstream to a coding sequence of an immunomodulatory factor for regulating the expression of the immunomodulatory factor. As used herein, the term "immunomodulatory factor" refers to a protein or small molecule polypeptide that can transmit information between cells and has immunomodulatory and effector functions. The promoter of the present application may be used to express an immunomodulatory factor selected from the group consisting of: cytokines, chemokines, antibodies, and chimeric antigen receptors, but not limited thereto. In one embodiment, the expressed cytokine may be: IL-2, IL-15, IL-12, IL-18, IL-21, IL-7, INF-α, IFN-β, IFN-γ, but not limited thereto. In one embodiment, the expressed chemokine may be: CCL20, CCL1, CCL17, CCL22, CCL27, CCL28, CXCL9, CXLC10, but not limited thereto. In one embodiment, the expressed antibody and/or chimeric antigen receptor targets the antigen selected from the group consisting of: PD1, 4-1BB, OX40, CD40, CTLA-4, CSF1R, and FAP, but not limited thereto. In one embodiment, the expressed immunomodulatory factor may also be NAP (neutrophil-activating protein) or MMPs (matrix metalloproteinases).

In one embodiment, the promoter of the present application is used to regulate the expression of cytokine. The promoter of the present application expresses substantially no or only a very small amount of the target protein when T cells are in inactive state, and upon T cell activation, can efficiently respond to T cell activation signal and induce a large amount of target protein expression. This performance can effectively enhance the activity and persistence of T cells and reduce the occurrence of side effects such as cytokine storms. Therefore, the promoter of the present application is suitable for CAR-T cells targeting tumors.

### Chimeric antigen receptor immune cell (CAR-immune cell)

In the present application, a chimeric antigen receptor immune cell is provided, which simultaneously expresses a chimeric antigen receptor and an immunomodulatory factor.

In the CAR-immune cell of the present application, the coding sequences of the chimeric antigen receptor and the immunomodulatory factor may be located in the same vector or located in different vectors, wherein the expression of the immunomodulatory factor is regulated by the promoter element of the present application, thereby expressing the immunomodulatory factor under the condition where the immune cells are activated by tumor antigens. In one embodiment, the CAR-immune cell contains the expression cassette for co-expressing the chimeric antigen receptor and the immunomodulatory factor in the third aspect of the present application.

It should be understood that the promoter or immunomodulatory factor expression cassette of the present application can be used in the immune cell expressing any chimeric antigen receptors, where these chimeric antigen receptors may target any known antigens (such as a tumor antigen). Therefore, while the present application provides examples of chimeric antigen receptors targeting B7-H3, the content of the present application should not be limited thereto.

The chimeric antigen receptor immune cell of the present application may be any immune cell, for example, may be CAR-T cell, may also be CAR-NK cell, or CAR-macrophage. Preferably, the chimeric antigen receptor immune cell of the present application is CAR-T cell.

As used herein, the terms "CAR-T cell", "CAR-T", and "CAR-T cell of the present application" all refer to the CAR-T cell in the sixth aspect of the present application.

The CAR-T cell has the following advantages over other T-cell-based therapy methods: (1) the mechanism of action of the CAR-T cell is not restricted by MHC; (2) given that many tumor cells express the same tumor markers, a CAR gene targeting a certain tumor marker can be widely used upon being constructed; (3) CAR can utilize both tumor protein markers and glycolipid non-protein markers, expanding the target range of tumor markers; (4) the use of the autologous cell of the patient reduces the risk of rejection reaction; and (5) CAR-T cell has immune memory function and can survive in the body for a long time.

As used herein, the terms "CAR-NK cell", "CAR-NK" and "CAR-NK cell of the present application" all refer to the CAR-NK cell in the sixth aspect of the present application. The CAR-NK cell of the present application may be used to treat tumors.

Natural killer (NK) cells are a major type of immune effector cells that protect the body from viral infection and tumor cell invasion through non-antigen-specific pathways. New function may be obtained by the engineered (genetically modified) NK cell, including the ability to specifically recognize tumor antigens and enhanced antitumor cytotoxicity.

Compared to CAR-T cells, the CAR-NK cells have the following advantages, for example: (1) they directly kill tumor cells by releasing perforin and granzymes, without killing normal cells in the body; (2) they release very few cytokines, thus reducing the risk of cytokine storms; and (3) they are very easy to expand in vitro and develop into a "ready-made" product. In addition to those, they are similar to CAR-T cell therapy.

### Vector

The present application also provides vectors containing the expression cassette of the present application. The vectors derived from retroviruses, such as lentiviruses, are suitable tools for achieving long-term gene transfer, because they allow for long-term stable integration of transgenes and their amplification in daughter cells. Lentiviral vectors have advantages over vectors derived from oncogenic retroviruses, such as murine leukemia viruses, because they can transduce non-proliferating cells, such as hepatocytes. They also have the advantage of low immunogenicity.

In brief, the promoter of the present application is typically operatively linked to the target nucleic acid sequence and incorporated into an expression vector. This vector is suitable for replication in and integration into eukaryotic cells. A typical cloning vector contains transcriptional and translational terminators, an initial sequence, and a promoter, for regulating the expression of the desired nucleic acid sequence.

The expression construct of the present application may also be used for nucleic acid immunization and gene therapy by using the standard gene delivery scheme. Methods of gene delivery are known in the art. See, for example, U.S. Pat. Nos. 5,399,346, 5,580,859, 5,589,466, which are incorporated herein by reference in their entirety. In another embodiment, the present application provides a gene therapy vector.

The expression cassette of the present application may be cloned into many types of vectors. For example, the expression cassette may be cloned into following vectors, including but not limited to, plasmids, phagemids, phage derivatives, animal viruses, and cosmids. Specific vectors of interest include expression vectors, replication vectors, probe generation vectors, and sequencing vectors.

Further, the expression vector may be provided to cells in the form of a viral vector. Viral vector technology is well known in the art and has been described in, for example, Sambrook et al. (2001, Molecular Cloning: A Laboratory Manual, Cold Spring Harbor Laboratory, New York) and other virology and molecular biology manuals. Viruses that may be used as vectors include, but are not limited to, retroviruses, adenoviruses, adeno-associated viruses, herpesviruses, and lentiviruses. Typically, a suitable vector contains a replication origin, a promoter sequence, a convenient restriction enzyme cutting site, and one or more selectable markers functioning in at least one organism (e.g., WO01/96584; WO01/29058; and U.S. Pat. No. 6,326,193).

Many virus-based systems have been developed for transferring genes into mammalian cells. For example, retroviruses provide a convenient platform for gene delivery systems. Selected genes may be inserted into vectors and packaged into retroviral particles by using techniques known in the art. Then the recombinant virus may be isolated and delivered to subject cells in vivo or ex vitro. Many retroviral systems are known in the art. In some embodiments, adenoviral vectors are used. Many adenoviral vectors are known in the art. In one embodiment, lentiviral vectors are used.

To assess the expression of CAR polypeptides or parts thereof, the expression vector introduced into the cell may also contain either or both of a selectable marker gene or a reporter gene, to facilitate the identification and selection of expression cells from a cell population seeking transfection or infection via a viral vector. In other aspects, the selectable marker may be carried on a single piece of DNA and used in co-transfection procedure. Both the selectable marker and the reporter gene may be flanked with appropriate regulatory sequences to be able to be expressed in host cells. Useful selectable markers include, for example, antibiotic resistance genes, such as neo.

The reporter gene is used to identify potentially transfected cells and to evaluate the functionality of regulatory sequences. Typically, the reporter gene is the following gene which is not present in the recipient organism or tissue or expressed by the recipient organism or tissue, and encodes a polypeptide whose expression is clearly represented by some easily detectable properties such as enzyme activity. The expression of the reporter gene is determined at an appropriate time after DNA has been introduced into the recipient cell. Suitable reporter gene may include the gene encoding luciferase, β-galactosidase, chloramphenicol acetyltransferase, secretory alkaline phosphatase, or green fluorescent protein (e.g., Ui-Tei et al., 2000 FEBS Letters 479:79-82). In one embodiment of the present application, the reporter gene is a gene encoding a Flag tag protein. Suitable expression systems are well known and may be prepared using known techniques or commercially available. Typically, a construct with at least 5 flanking regions that exhibits the highest level of reporter gene expression is identified as a promoter. Such promoter region may be linked to the reporter gene and used to evaluate the ability of a reagent regulating promoter-driven transcription.

Methods for introducing genes into cells and expressing genes into cells are known in the art. In the content of the expression vector, the vector may be easily introduced into a host cell, e.g., mammalian, bacteria, yeast, or insect cells, by any method in the art. For example, the expression vector may be transferred into the host cell by physical, chemical, or biological means.

Physical methods for introducing polynucleotides into host cells include calcium phosphate precipitation, lipid transfection, particle bombardment, microinjection, electroporation, etc. Methods for producing cells including vectors and/or exogenous nucleic acids are well known in the art. See, for example, Sambrook et al. (2001, Molecular Cloning: A Laboratory Manual, Cold Spring Harbor Laboratory, New York). A preferred method for introducing polynucleotides into host cells is calcium phosphate transfection.

Biological approaches for introducing polynucleotides of interest into host cells include the use of DNA and RNA vectors. Viral vectors, particularly retroviral vectors, have become the most widely used method for inserting genes into mammalian cells, such as human cells. Other viral vectors may be derived from lentiviruses, poxviruses, herpes simplex virus I, adenoviruses, and adeno-associated viruses, etc. See, for example, U.S. Patent Nos. 5,350,674 and 5,585,362.

Chemical means of introducing polynucleotides into host cells include colloidal dispersion systems, such as macromolecular complexes, nanocapsules, microspheres, and beads; and lipid-based systems, including oil-in-water emulsions, micelles, mixed micelles, and liposomes. An exemplary colloidal system used as a delivery vehicle both in vitro and in vivo is a liposome (e.g., an artificial membrane capsule).

In the case of using a non-viral delivery system, an exemplary delivery vehicle is liposome. The lipid formulation is considered to be used to introduce nucleic acids into host cells (in vitro, ex vivo, or in vivo). In another aspect, the nucleic acid may be associated with a lipid. The lipid-associated nucleic acid may be encapsulated into the aqueous interior of the liposome, scattered in the lipid bilayer of the liposome, attached to the liposome via a linker molecule associated with both the liposome and the oligonucleotide, trapped within the liposome, compounded with the liposome, dispersed in a solution containing lipids, mixed with the lipids, combined with the lipids, contained in the lipids as suspension, contained in micelle or compounded with micelle, or otherwise associated with lipids. The lipids, lipid/DNA, or lipid/expression vector associated with the composition are not limited to any specific structure in the solution. For example, they may be present in a bilayer structure, as the micelle, or have a "collapsed" structure. They may also be simply scattered in the solution, possibly forming aggregates of uneven sizes or shapes. Lipids are fatty substances and may be naturally occurring or synthetic lipids. For example, the lipids include fat droplets which occur naturally in the cytoplasm and in compounds containing long-chain aliphatic hydrocarbons and their derivatives such as fatty acids, alcohols, amines, amino alcohols and aldehydes.

In a preferred embodiment of the present application, the vector is a retroviral vector.

### Formulation

The present application provides a formulation including the vector in the fourth aspect of the present application, or the host cell in the fifth aspect of the present application, or the engineered immune cell in the sixth aspect of the present application, and a pharmaceutically acceptable carrier, diluent, or excipient. In one embodiment, the formulation is a liquid formulation. Preferably, the formulation is an injection. Preferably, the concentration of the CAR-T cell in the formulation is 1×10³-1×10⁸ cells/ml, preferably 1×10⁴-1×10⁷ cells/ml.

In one embodiment, the formulation may include a buffer solution such as neutral buffered saline, sulfate buffered saline, etc.; a carbohydrate such as glucose, mannose, sucrose or glucan, mannitol; protein; polypeptide or amino acid such as glycine; antioxidant; chelating agent such as EDTA or glutathione; adjuvant (e.g., aluminum hydroxide); and preservative. The formulation of the present application is preferably formulated for intravenous administration.

### Therapeutic use

The present application includes a therapeutic use performed using the cell (e.g., T cell) transduced with a vector encoding the expression cassette of the present application. The transduced T cell may target tumor cell markers (such as B7-H3), and induce the expression of immunomodulatory factors after T cell activation, resulting in sustained T cell activation and immune response, thereby significantly improving the killing efficiency thereof against tumor cells.

Therefore, the present application also provides a method for stimulating a T cell-mediated immune response against a target cell population or tissue in mammal, which includes the following step: administering the CAR-cell of the present application to a mammal.

In one embodiment, the present application includes a type of cell therapy in which autologous T cells from a patient (or allogeneic cells from a heterologous donor) T cells are isolated, activated, and genetically modified to produce CAR-T cells, which are then injected into the same patient. For this method, the probability of having graft-versus-host disease is extremely low, and the antigen is recognized by the T cells in an MHC-free manner. Furthermore, a kind of CAR-T cell can treat all cancers expressing that antigen. Unlike antibody therapy, CAR-T cells can replicate in vivo, resulting in long-term persistence that can lead to sustained tumor control.

In one embodiment, the CAR-T cells of the present application can undergo stable in vivo T cell expansion and persist for an extended period of time. Additionally, the CAR-mediated immune response can be part of an adoptive immunotherapy step, where the CAR-modified T cells induce an immune response specific to the antigen-binding domain in the CAR. For example, the CAR-T cells expressing B7-H3 CAR elicit a specific immune response against B7-H3 cells.

Although the data disclosed herein specifically discloses the retroviral vector including a combination of anti-B7-H3 scFv, hinge and transmembrane regions, co-stimulatory domain of CD28, and CD3ζ signaling domain, the present application should be construed as including any number of variations in each of the construct components.

Treatable cancers include tumors that are not vascularized or substantially not vascularized, and vascularized tumors. The cancers include non-solid tumors (such as hematologic malignancies, e.g., leukemia and lymphoma) and solid tumors. Types of cancers treated with the CAR of the present application include, but are not limited to, carcinoma, blastocytoma, and sarcomas, and certain leukemias or lymphoid malignancies, benign and malignant tumors, and malignant tumors such as sarcomas, carcinomas, and melanomas. Adult tumors/cancers and pediatric tumors/cancers are also included.

Hematologic cancers are cancers of the blood or bone marrow. Examples of hematologic (or hemogenic) cancers include leukemia, including acute leukemia (such as acute lymphoblastic leukemia, acute myeloid leukemia, acute myeloid leukemia, and myeloblastic leukemia, promyelocytic leukemia, granulo-monocytic leukemia, monocytic leukemia, and erythroleukemia), chronic leukemia (such as chronic myeloid (granulocytic) leukemia, chronic myeloid leukemia, and chronic lymphocytic leukemia), polycythemia vera, lymphoma, Hodgkin's disease, non-Hodgkin's lymphoma (painless and high-grade forms), multiple myeloma, Waldenström's macroglobulinemia, heavy chain disease, myelodysplastic syndrome, hairy cell leukemia, and spinal cord dysplasia.

The CAR-modified T cells of the present application may also be used as a type of vaccine for ex vivo immunization and/or in vivo therapy in mammals. Preferably, the mammal is human.

For ex vivo immunization, at least one of the following occurs in vitro before the cells are administered into a mammal: i) expanding the cells, ii) introducing CAR-encoding nucleic acid into the cells, and/or iii) cryopreserving the cells.

The ex vivo procedure is well known in the art and discussed more fully below. In brief, cells are isolated from a mammal (preferably human) and genetically modified (i.e., transduced or transfected in vitro) with the vector expressing the CAR disclosed herein. CAR-modified cells may be administered to a mammalian recipient to provide therapeutic benefits. The mammalian recipient may be human, and CAR-modified cells may be autologous relative to the recipient. Optionally, the cells may be allogeneic, syngeneic or xenogeneic relative to the recipient.

In addition to the use of cell-based vaccines for ex vivo immunization, the present application also provides compositions and methods for in vivo immunization to induce an immune response against an antigen in a patient.

The present application provides a method for treating a tumor, including administering a therapeutically effective amount of the CAR-modified T cell of the present application to a subject in need thereof.

The CAR-modified T cell of the present application may be administered alone or used as a pharmaceutical composition in combination with a diluent and/or other component such as IL-2, IL-17, or other cytokine or cell population. Briefly, the pharmaceutical compositions of the present application may include the target cell population as described herein, combined with one or more pharmaceutically or physiologically acceptable carriers, diluents, or excipients. Such composition may include a buffer such as neutral buffered saline, sulfate buffered saline, etc.; a carbohydrate such as glucose, mannose, sucrose or glucan, mannitol; protein; polypeptide or amino acid such as glycine; antioxidant; chelating agent such as EDTA or glutathione; adjuvant (e.g., aluminum hydroxide); and preservative. The composition of the present application is preferably formulated for intravenous administration.

The pharmaceutical composition of the present application may be administered in a manner suitable for the disease to be treated (or prevented). The amount and frequency of administration may be determined by such factors as the patient's condition, and the type and severity of the patient's disease - although the appropriate dosage may be determined by clinical trials.

When referring to "effective amount", "immunologically effective amount", "antitumor effective amount", "tumor-suppressive effective amount" or "therapeutic amount", the precise amount of the composition of the present application to be administered may be determined by a physician, who takes into account individual differences in the age, weight, tumor size, degree of infection or metastasis, and condition of the patient (subject). It may generally be indicated that: the pharmaceutical composition including the T cell described herein may be administered at a dose of 10⁴ to 10⁹ cells/kg body weight, preferably 10⁵ to 10⁶ cells/kg body weight (including all integer values in those ranges). The T cell composition may also be administered multiple times at these doses. Cells may be administered by using injection techniques well known in immunotherapy (see, for example, Rosenberg et al., New Eng. J. of Med. 319:1676, 1988). The optimal dose and treatment regimen for a particular patient may be determined easily by those skilled in the medical field by monitoring the disease signs of the patient and adjusting the treatment accordingly.

The administration of the composition to a subject may be carried out in any convenient manner, including by spraying, injection, swallowing, infusion, implantation or transplantation. The composition described herein may be administered to a patient subcutaneously, intradermally, intratumorally, intranodally, intraspinally, intramuscularly, by intravenous (i.v.) injection, or intraperitoneally. In one embodiment, the T cell composition of the present application is administered to a patient by intradermal or subcutaneous injection. In another embodiment, the T cell composition of the present application is preferably administered by i.v. injection. The composition of T cells may be injected directly into the tumor, lymph node or infected site.

In certain embodiments of the present application, cells activated and expanded using the methods described herein or other methods for expanding cells to a therapeutic level known in the art are administered to the patient (e.g., before, together with, or after) combination with any number of relevant treatment forms, where the treatment forms includes but not limited to treatment with the following agents: the agents such as antiviral therapy, cidofovir and interleukin-2, cytarabine (also known as ARA-C) or natalizumab therapy in MS patient or efalizumab therapy in psoriasis patient or other therapy for specific tumor patients. In further embodiments, the T cell of the present application may be used in combination with the followings: chemotherapy, radiation, immunosuppressant such as cyclosporin, azathioprine, methotrexate, mycophenolate and FK506, antibody or other immunotherapeutic agent. In further embodiment, the cell composition of the present application is administered to a patient (e.g., before, together with, or after) combination with bone marrow transplantation, the use of a chemotherapeutic agent such as fludarabine, external beam radiotherapy (XRT), cyclophosphamide. For example, in one embodiment, a subject may undergo a standard treatment of high-dose chemotherapy followed by peripheral blood stem cell transplantation. In some embodiments, after transplantation, the subject receives an injection of the expanded immune cells of the present application. In an additional embodiment, the expanded cells are administered before surgery or after surgery.

The above treatment doses administered to patients may vary depending on the precise nature of the treated condition and the treatment recipient. The dose ratio administered to a person may be implemented according to practices accepted in the art. Typically, 1×10⁶ to 1×10¹⁰ CAR-T cells of the present application may be administered to the patient per treatment or per course of treatment, for example, via intravenous reinfusion.

**The main advantages of the present application include the follows.**
1) The present application provides a series of novel T cell activation induced recombinant promoters constructed by modifying and optimizing GM-CSF, IFNg and IL2 promoters, which can efficiently induce the expression of their downstream genes when CAR-T cells are activated, and their activity is superior to that of traditional NFAT-responsive promoters constructed based on the NFAT (nuclear factor of activated T cells) binding sequence.
2) The present application provides the CAR-T cell containing the T cell activation induced recombinant promoter, which can induce the CAR-T cell to secrete and express various immunomodulatory molecules such as cytokines and antibody-like proteins in tumors, to promote the activity and persistence of CAR-T cells and thus promote the application of the CAR-T cells in solid tumors.

The present application will be further illustrated below with reference to specific examples. It should be understood that these examples are merely for illustrate the present application and not intended to limit the scope of the present application. Experimental methods in the following examples that do not specify specific conditions are generally performed based on conventional conditions, e.g., the conditions described by Sambrook et al. Molecular Cloning: A Laboratory Manual (New York: Cold Spring Harbor Laboratory Press, 1989) or conditions as suggested by the manufacturers. Unless otherwise stated, percentages and parts are weight percentages and parts by weight.

### Example 1: Construction of different versions of B7H3.28ζ.TAIP.mIL2 plasmid

Different fragments of the GM-CSF, IFNg, and IL2 promoters were selected to construct T cell activation induced promoters (TAIPs), with the mIL2 gene inserted thereafter as an indicator gene (reporter gene). TAIP-mIL2 was then cloned in the reverse orientation into the SFG.B7H3.28ζ expression vector, with a bidirectional polyA (PA2) sequence interposed therebetween, constructing a series of B7H3.28ζ.TAIP.mIL2 vectors (as shown in FIG. 1). In the recombinant promoter of the GM-CSF promoter and the minCMV minimal promoter, multiple versions of the recombinant promoters were constructed with minCMV located at different downstream positions of the GM-CSF promoter (pGM-CSF) (as shown in FIG. 1), to enhance the induction activity of pGM-CSF. In the above, SFG.B7H3.28z.CAR was used as a negative control vector, and B7H3.28ζ.NFAT.mIL2 was used as a reference vector.

The construction method was as follows: the fragment sequences of light-chain and heavy-chain variable regions (VL and VH) of the anti-B7H3 monoclonal antibody 376.96 were synthesized by using a gene sequence synthesis method, digested with MluI and NcoI enzymes, and then cloned into the retroviral vector SFG.CAR.CD28ζ to construct the SFG.B7H3.CD28ζ vector. Different fragments of the GM-CSF, IFNg, and IL2 promoters were amplified from the genome of human cells by using PCR. The mIL2 gene was amplified from the genome of activated mouse T cells. These two were then linked through a fusion method and cloned in the reverse orientation into the SFG.B7H3.CD28ζ vector. In a plasmid containing minCMV, minCMV was introduced downstream of the GM-CSF promoter fragment by using primers and then cloned into the SFG.B7H3.CD28ζ vector together with the mIL2 gene by using the fusion method.

### Example 2: Preparation of retrovirus expressing B7H3.28ζ.TAIP.mIL2

6×10⁵ 293T cells were inoculated into a 6-well cell culture plate. After 18 h, 0.75 µg of different versions of B7H3.28ζ.TAIP.mIL2 plasmids were mixed with 0.75 µg of PEQ plasmid and 0.5 µg of RDF plasmid respectively. Then the plasmid mixture was then mixed with GeneJuice transfection reagent (Merck Millipore), stood for 10 min, and then transfected into 293T cells. 48 h after transfection, the virus-containing culture supernatant was collected, filtered through a 0.45 µm filter, and then cryopreserved at -80°C for later use.

### Example 3: Preparation and in vitro expansion of CAR-T cell

PBMC lymphocytes were isolated from the peripheral blood of healthy volunteers and then added to 24-well plates coated with CD3 and CD28 antibodies, at 1×10⁶ cells per well. After 24 hours, IL-7 and IL-15 (5 ng/mL) were added, and T cells were collected after two days of stimulation and counted for later use. The stimulated T cells were infected with the aforementioned virus. The specific infection steps were as follows: 1 mL of viral supernatant was taken and added to a 24-well plate coated with fibronectin and centrifuged at 2000 g for 1.5 h. After centrifugation, the supernatant was discarded, and 3×10⁵ stimulated T cells were added and centrifuged at 1000 g for 10 min. The cells were cultured in a 37°C cell culture incubator, and the medium was replaced with fresh medium after 72 h. Thereafter, the medium was replaced every two days. 4 days after infection, CAR-T cells were collected, and stained using anti-Flag antibody, and the CAR positivity rate was detected by flow cytometry detection. 7 days after infection, 3×10⁵ cells were collected for B7H3-FC antigen stimulation experiment.

### Example 4 Flow Cytometry Detection

4 days after viral infection, 1×10⁶ CAR-T cells were collected and stained, added with 0.5 µg of Flag-APC (Biolegend) antibody, and incubated at 4°C for 15 min. The cells were washed with PBS and detected by BD FACSCanto II flow cytometer. At least 10,000 live cells were collected for each sample, and the collected results were analyzed using Flowjo 10 software.

Results: B7H3.28ζ-TAIP-mIL2 may be efficiently expressed in T cells (as shown in FIG. 2). FIG. 2B and FIG. 2C show that the positivity rate and expression level of the chimeric antigen receptor were not significantly affected, which basically met the requirements for use.

### Example 5 B7H3-FC antigen stimulation experiment

B7H3-FC protein (ACROBiosystem) was diluted in PBS to a final concentration of 1 µg/ml. 500 µl thereof was then collected and added to a 24-well non-tissue culture plate, and placed at 4°C for coating overnight. The next day, the B7H3-FC diluted solution was discarded, and 1 mL of serum-containing culture medium was added. The plate was incubated at a room temperature for 10 min, and then 1×10⁶ CAR-T cells were seeded into each well, and simultaneously, the same number of CAR-T cells were seeded into the well of a 24-well plate uncoated with B7H3-FC, as an unstimulated control group. After incubation at 37°C for 24 h, 1 mL of supernatant was collected for ELISA detection. The secretion level of mIL2 in the supernatant of B7H3.28ζ.TAIP.mIL2-infected T cells after B7H3-FC stimulation was detected by using a mouse IL2 ELISA kit (Mabtech).

Results: as shown in FIG. 3, after 24 hours of antigen stimulation, the series of inducible promoters constructed all can responded to T cell activation and induce the expression and secretion of mIL2. Except the pIFN (+64 to -108) promoter, the rest inducible promoters show higher induction activity than the NFAT promoter. Among them, the recombinant promoters composed of the GM-CSF-derived promoters and mimCMV (pGM-CSF(-1 to -336).minCMV, pGM-CSF(-35 to -336).minCMV, and pGM-CSF(-1 to -217).minCMV) can efficiently induce mIL2 expression. The quantitative results are as shown in Table 1 below:

**Table 1 mIL2 expression level**

| Promoter name | After B7H3-FC stimulation | | No stimulation (pg/ml/10⁶ cells) |
|---|---|---|---|
| | Absolute expression (pg/ml/10⁶ cells) | Relative expression | |
| pGM-CSF(-35 to - 336).minCMV | 8670.36 | 8.03 | 222.67 |
| pGM-CSF(-1 to - 217).minCMV | 8437.81 | 8.69 | 224.72 |
| pGM-CSF(+36 to - 217).minCMV | 7129.81 | 7.40 | 149.00 |
| pGM-CSF (-1 to -336).minCMV | 5559.10 | 4.91 | 229.77 |
| pGM-CSF(+36 to - 336).minCMV | 4276.11 | 3.89 | 186.12 |
| pGM-CSF (+36 to -217) | 4332.30 | 3.54 | 64.45 |
| pGM-CSF (+36 to -626) | 3684.14 | 2.77 | 114.63 |
| pGM-CSF (-1 to -217) | 3508.93 | 3.02 | 105.10 |
| pGM-CSF (+36 to -336) | 3007.35 | 2.69 | 48.43 |
| pGM-CSF (-1 to -336) | 2544.46 | 2.49 | 76.71 |
| pIL2 (+47 to -326) | 2716.73 | 1.92 | 4.04 |
| pIFN (+64 to -538) | 5597.77 | 5.10 | 361.72 |
| pIFN (+64 to -108) | 1053.54 | 1.06 | 130.14 |
| NFAT (SEQ ID NO: 30) | 1502.96 | 1.00 | 3.10 |
| B7H3. 28ζ | -0.89 | / | -4.16 |

The results show that when minCMV is located 35 bp downstream of pGM-CSF, the pGM-CSF(-35 to -336).minCMV promoter is stronger than the pGM-CSF(-1 to -336).minCMV (as shown in FIG. 3). The induction activities of pGM-CSF(-35 to -217).minCMV and pGM-CSF(-1 to -217).minCMV were then detected and compared. The results show that the induction activity of the pGM-CSF(-35 to -217).minCMV is slightly better than that of the pGM-CSF(-1 to -217).minCMV (as shown in FIG. 4).

### Example 6 Promoter truncation test

The GM-CSF promoter fragment was further truncated to test whether the shorter GM-CSF promoter still possessed strong T cell activation induction activity. The results are as shown in FIG. 5. The further truncated pGM-CSF(-35 to -179).minCMV still retain the induction activity comparable to that of the pGM-CSF(-35 to -217).minCMV. However, when the promoter fragment is shortened to approximately 100 bp, for example, the pGM-CSF(-35 to -106).minCMV promoter, the induction activity thereof is significantly lower than that of the pGM-CSF(-35 to - 217).minCMV, but still higher than that of the synthetic promoter of hexameric repeat of the NFAT binding sequence (FIG. 5).

### Sequences:

1. DNA sequence of 376.96 scFv: 
GACATTGTGATGACCCAGTCTCACAAATTCATGTCCACATCAATTGGAGCCA
GGGTCAGCATCACCTGCAAGGCCAGTCAGGATGTGAGAACTGCTGTAGCCTGGTATC
AACAGAAACCAGGCCAGTCTCCTAAACTACTAATTTACTCGGCATCCTACCGGTACA
CTGGAGTCCCTGATCGCTTCACTGGCAGTGGATCTGGGACGGATTTCACTTTCACCAT
CAGCAGTGTGCAGGCTGAAGACCTGGCAGTTTATTACTGTCAGCAACATTATGGTAC
TCCTCCGTGGACGTTCGGTGGAGGCACCAAGCTGGAAATCAAAGGCGGCGGAGGAT
CTGGCGGAGGCGGAAGTGGCGGAGGGGGCTCTGAAGTGCAGCTGGTGGAGTCTGG
GGGAGGCTTAGTGAAGCCTGGAGGGTCCCTGAAACTCTCCTGTGAAGCCTCTAGATT
CACTTTCAGTAGCTATGCCATGTCTTGGGTTCGCCAGACTCCGGAGAAGAGGCTGGA
GTGGGTCGCAGCCATTAGTGGAGGTGGTAGGTACACCTACTATCCAGACAGTATGAA
GGGTCGATTCACCATCTCCAGAGACAATGCCAAGAATTTCCTGTACCTGCAAATGAG
CAGTCTGAGGTCTGAGGACACGGCCATGTATTACTGTGCAAGACACTATGATGGTTAT
CTTGACTACTGGGGCCAAGGCACCACTCTCACAGTCTCCTCA (SEQ ID NO: 1)
2. Amino acid sequence of 376.96 scFv: 
DIVMTQSHKFMSTSIGARVSITCKASQDVRTAVAWYQQKPGQSPKLLIYSASYR
YTGVPDRFTGSGSGTDFTFTISSVQAEDLAVYYCQQHYGTPPWTFGGGTKLEIKGGGGS
GGGGSGGGGSEVQLVESGGGLVKPGGSLKLSCEASRFTFSSYAMSWVRQTPEKRLEWV
AAISGGGRYTYYPDSMKGRFTISRDNAKNFLYLQMSSLRSEDTAMYYCARHYDGYLDY
WGQGTTLTVSS (SEQ ID NO: 2)
3. DNA sequence of CD8α hinge region and transmembrane region: 
ACCACGACGCCAGCGCCGCGACCACCAACACCGGCGCCCACCATCGCGTC
GCAGCCCCTGTCCCTGCGCCCAGAGGCGTGCCGGCCAGCGGCGGGGGGCGCAGTGC
 
ACACGAGGGGGCTGGACTTCGCCTGTGATATCTACATCTGGGCGCCCTTGGCCGGGA
CTTGTGGGGTCCTTCTCCTGTCACTGGTTATCACCCTTTACTGC (SEQ ID NO: 3)
4. Amino acid sequence of the CD8α hinge region and transmembrane region: 
TTTPAPRPPTPAPTIASQPLSLRPEACRPAAGGAVHTRGLDFACDIYIWAPLAGT
CGVLLLSLVITLYC (SEQ ID NO: 4)
5. DNA sequence of CD28 intracellular co-stimulatory domain: 
AGGAGTAAGAGGAGCAGGCTCCTGCACAGTGACTACATGAACATGACTCCC
CGCCGCCCCGGGCCCACCCGCAAGCATTACCAGCCCTATGCCCCACCACGCGACTTC
GCAGCCTATCGCTCC (SEQ ID NO: 5)
6. Amino acid sequence of CD28 intracellular co-stimulatory domain:
   RSKRSRLLHSDYMNMTPRRPGPTRKHYQPYAPPRDFAAYRS (SEQ ID NO: 6)
7. DNA sequence of CD3ζ intracellular domain: 
AGAGTGAAGTTCAGCAGGAGCGCAGACGCCCCCGCGTACCAGCAGGGCCA
GAACCAGCTCTATAACGAGCTCAATCTAGGACGAAGAGAGGAGTACGATGTTTTGGA
CAAGAGACGTGGCCGGGACCCTGAGATGGGGGGAAAGCCGAGAAGGAAGAACCCT
CAGGAAGGCCTGTACAATGAACTGCAGAAAGATAAGATGGCGGAGGCCTACAGTGA
GATTGGGATGAAAGGCGAGCGCCGGAGGGGCAAGGGGCACGATGGCCTTTACCAGG
GTCTCAGTACAGCCACCAAGGACACCTACGACGCCCTTCACATGCAGGCCCTGCCCC
CTCGC (SEQ ID NO: 7)
8. Amino acid sequence of CD3ζ intracellular domain: 
RVKFSRSADAPAYQQGQNQLYNELNLGRREEYDVLDKRRGRDPEMGGKPRR
KNPQEGLYNELQKDKMAEAYSEIGMKGERRRGKGHDGLYQGLSTATKDTYDALHMQA
LPPR (SEQ ID NO: 8)
9. DNA sequence of mouse IL2 (mIL2) CDS: 
ATGTACAGCATGCAGCTCGCATCCTGTGTCACATTGACACTTGTGCTCCTTG
TCAACAGCGCACCCACTTCAAGCTCCACTTCAAGCTCTACAGCGGAAGCACAGCAG
CAGCAGCAGCAGCAGCAGCAGCAGCAGCAGCACCTGGAGCAGCTGTTGATGGACC
TACAGGAGCTCCTGAGCAGGATGGAGAATTACAGGAACCTGAAACTCCCCAGGATG
CTCACCTTCAAATTTTACTTGCCCAAGCAGGCCACAGAATTGAAAGATCTTCAGTGC
CTAGAAGATGAACTTGGACCTCTGCGGCATGTTCTGGATTTGACTCAAAGCAAAAGC
 
TTTCAATTGGAAGATGCTGAGAATTTCATCAGCAATATCAGAGTAACTGTTGTAAAAC
TAAAGGGCTCTGACAACACATTTGAGTGCCAATTCGATGATGAGTCAGCAACTGTGG
TGGACTTTCTGAGGAGATGGATAGCCTTCTGTCAAAGCATCATCTCAACAAGCCCTC
AATAA (SEQ ID NO: 9)
10. Amino acid sequence of mouse IL2 (mIL2) protein: 
MYSMQLASCVTLTLVLLVNSAPTSSSTSSSTAEAQQQQQQQQQQHLEQLL
MDLQELLSRMENYRNLKLPRMLTFKFYLPKQATELKDLQCLEDELGPLRHVLDLTQSK
SFQLEDAENFISNIRVTVVKLKGSDNTFECQFDDESATVVDFLRRWIAFCQSIISTSPQ*
(SEQ ID NO: 10)
11. DNA sequence of pGM-CSF (+36 to -336) promoter 
TAAGTGTCTCCCACGCCCCACCCCAGCCATTCCAGGCCAGGAAGTCCAAAC
TGTGCCCCTCAGAGGGAGGGGGCAGCCTCAGGCCCATTCAGACTGCCCAGGGAGGG
CTGGAGAGCCCTCAGGAAGGCGGGTGGGTGGGCTGTCGGTTCTTGGAAAGGTTCAT
TAATGAAAACCCCCAAGCCTGACCACCTAGGGAAAAGGCTCACCGTTCCCATGTGTG
GCTGATAAGGGCCAGGAGATTCCACAGTTCAGGTAGTTCCCCCGCCTCCCTGGCATT
TTGTGGTCACCATTAATCATTTCCTCTGTGTATTTAAGAGCTCTTTTGCCAGTGAGCCC
AGTACACAGAGAGAAAGGCTAAAGTTCTCTGGAGGA (SEQ ID NO: 11)
12. DNA sequence of pGM-CSF (-1 to -336) promoter 
TAAGTGTCTCCCACGCCCCACCCCAGCCATTCCAGGCCAGGAAGTCCAAAC
TGTGCCCCTCAGAGGGAGGGGGCAGCCTCAGGCCCATTCAGACTGCCCAGGGAGGG
CTGGAGAGCCCTCAGGAAGGCGGGTGGGTGGGCTGTCGGTTCTTGGAAAGGTTCAT
TAATGAAAACCCCCAAGCCTGACCACCTAGGGAAAAGGCTCACCGTTCCCATGTGTG
GCTGATAAGGGCCAGGAGATTCCACAGTTCAGGTAGTTCCCCCGCCTCCCTGGCATT
TTGTGGTCACCATTAATCATTTCCTCTGTGTATTTAAGAGCTCTTTTGCCAGTGAGCCC
(SEQ ID NO: 12)
13. DNA sequence of pGM-CSF (-35 to -336) promoter 
TAAGTGTCTCCCACGCCCCACCCCAGCCATTCCAGGCCAGGAAGTCCAAAC
TGTGCCCCTCAGAGGGAGGGGGCAGCCTCAGGCCCATTCAGACTGCCCAGGGAGGG
CTGGAGAGCCCTCAGGAAGGCGGGTGGGTGGGCTGTCGGTTCTTGGAAAGGTTCAT
TAATGAAAACCCCCAAGCCTGACCACCTAGGGAAAAGGCTCACCGTTCCCATGTGTG
 
GCTGATAAGGGCCAGGAGATTCCACAGTTCAGGTAGTTCCCCCGCCTCCCTGGCATT
TTGTGGTCACCATTAATCATTTCCT (SEQ ID NO: 13)
14. DNA sequence of pGM-CSF (+36 to -217) promoter 
CAGGAAGGCGGGTGGGTGGGCTGTCGGTTCTTGGAAAGGTTCATTAATGAA
AACCCCCAAGCCTGACCACCTAGGGAAAAGGCTCACCGTTCCCATGTGTGGCTGATA
AGGGCCAGGAGATTCCACAGTTCAGGTAGTTCCCCCGCCTCCCTGGCATTTTGTGGT
CACCATTAATCATTTCCTCTGTGTATTTAAGAGCTCTTTTGCCAGTGAGCCCAGTACA
CAGAGAGAAAGGCTAAAGTTCTCTGGAGGA (SEQ ID NO: 14)
15. DNA sequence of pGM-CSF (-1 to -217) promoter 
CAGGAAGGCGGGTGGGTGGGCTGTCGGTTCTTGGAAAGGTTCATTAATGAA
AACCCCCAAGCCTGACCACCTAGGGAAAAGGCTCACCGTTCCCATGTGTGGCTGATA
AGGGCCAGGAGATTCCACAGTTCAGGTAGTTCCCCCGCCTCCCTGGCATTTTGTGGT
CACCATTAATCATTTCCTCTGTGTATTTAAGAGCTCTTTTGCCAGTGAGCCC (SEQ ID
NO: 15)
16. DNA sequence of pGM-CSF (-35 to -217) promoter 
CAGGAAGGCGGGTGGGTGGGCTGTCGGTTCTTGGAAAGGTTCATTAATGAA
AACCCCCAAGCCTGACCACCTAGGGAAAAGGCTCACCGTTCCCATGTGTGGCTGATA
AGGGCCAGGAGATTCCACAGTTCAGGTAGTTCCCCCGCCTCCCTGGCATTTTGTGGT
CACCATTAATCATTTCCT (SEQ ID NO: 16)
17. DNA sequence of pGM-CSF (+36 to -626) promoter 
GCTTGCTGAGAGTGGCTGCAGTCTCGCTGCTGGATGTGCACATGGTGGTCA
TTCCCTCTGCTCACAGGGGCAGGGGTCCCCCCTTACTGGACTGAGGTTGCCCCCTGC
TCCAGGTCCTGGGTGGGAGCCCATGTGAACTGTCAGTGGGGCAGGTCTGTGAGAGC
TCCCCTCACACTCAAGTCTCTCACAGTGGCCAGAGAAGAGGAAGGCTGGAGTCAGA
ATGAGGCACCAGGGCGGGCATAGCCTGCCCAAAGGCCCCTGGGATTACAGGCAGGA
TGGGGAGCCCTATCTAAGTGTCTCCCACGCCCCACCCCAGCCATTCCAGGCCAGGAA
GTCCAAACTGTGCCCCTCAGAGGGAGGGGGCAGCCTCAGGCCCATTCAGACTGCCC
AGGGAGGGCTGGAGAGCCCTCAGGAAGGCGGGTGGGTGGGCTGTCGGTTCTTGGA
AAGGTTCATTAATGAAAACCCCCAAGCCTGACCACCTAGGGAAAAGGCTCACCGTT
CCCATGTGTGGCTGATAAGGGCCAGGAGATTCCACAGTTCAGGTAGTTCCCCCGCCT
 
CCCTGGCATTTTGTGGTCACCATTAATCATTTCCTCTGTGTATTTAAGAGCTCTTTTGC
CAGTGAGCCCAGTACACAGAGAGAAAGGCTAAAGTTCTCTGGAGGA (SEQ ID NO: 17)
18. DNA sequence of pIL2 (+47 to -326) promoter 
TTTTCTGAGTTACTTTTGTATCCCCACCCCCTTAAAGAAAGGAGGAAAAACT
GTTTCATACAGAAGGCGTTAATTGCATGAATTAGAGCTATCACCTAAGTGTGGGCTAA
TGTAACAAAGAGGGATTTCACCTACATCCATTCAGTCAGTCTTTGGGGGTTTAAAGA
AATTCCAAAGAGTCATCAGAAGAGGAAAAATGAAGGTAATGTTTTTTCAGACAGGTA
AAGTCTTTGAAAATATGTGTAATATGTAAAACATTTTGACACCCCCATAATATTTTTCC
AGAATTAACAGTATAAATTGCATCTCTTGTTCAAGAGTTCCCTATCACTCTCTTTAATC
ACTACTCACAGTAACCTCAACTCCTGCCACA (SEQ ID NO: 18)
19. DNA sequence of pIFN (+64 to -538) promoter 
TAGAGCAATTTGAAACTTGTGGTAGATATTTTACTAACCAACTCTGATGAAG
GACTTCCTCACCAAATTGTTCTTTTAACCGCATTCTTTCCTTGCTTTCTGGTCATTTGC
AAGAAAAATTTTAAAAGGCTGCCCCTTTGTAAAGGTTTGAGAGGCCCTAGAATTTCG
TTTTTCACTTGTTCCCAACCACAAGCAAATGATCAATGTGCTTTGTGAATGAAGAGTC
AACATTTTACCAGGGCGAAGTGGGGAGGTACAAAAAAATTTCCAGTCCTTGAATGGT
GTGAAGTAAAAGTGCCTTCAAAGAATCCCACCAGAATGGCACAGGTGGGCATAATG
GGTCTGTCTCATCGTCAAAGGACCCAAGGAGTCTAAAGGAAACTCTAACTACAACA
CCCAAATGCCACAAAACCTTAGTTATTAATACAAACTATCATCCCTGCCTATCTGTCAC
CATCTCATCTTAAAAAACTTGTGAAAATACGTAATCCTCAGGAGACTTCAATTAGGTA
TAAATACCAGCAGCCAGAGGAGGTGCAGCACATTGTTCTGATCATCTGAAGATCAGC
TATTAGAAGAGAAAGATCAGTTAAGTCCTTTGG (SEQ ID NO: 19)
20. DNA sequence of pIFN (+64 to -108) promoter 
CTATCATCCCTGCCTATCTGTCACCATCTCATCTTAAAAAACTTGTGAAAATA
CGTAATCCTCAGGAGACTTCAATTAGGTATAAATACCAGCAGCCAGAGGAGGTGCAG
CACATTGTTCTGATCATCTGAAGATCAGCTATTAGAAGAGAAAGATCAGTTAAGTCCT
TTGG (SEQ ID NO: 20)
21. DNA sequence of minCMV (minimal CMV promoter)
   CCTATATAAGCAGAGCTCGTTTAGTGAACCGTCAGATCGCCTGGAGA (SEQ ID NO: 21)
22. DNA sequence of pGM-CSF(+36 to -336).minCMV promoter 
TAAGTGTCTCCCACGCCCCACCCCAGCCATTCCAGGCCAGGAAGTCCAAAC
TGTGCCCCTCAGAGGGAGGGGGCAGCCTCAGGCCCATTCAGACTGCCCAGGGAGGG
CTGGAGAGCCCTCAGGAAGGCGGGTGGGTGGGCTGTCGGTTCTTGGAAAGGTTCAT
TAATGAAAACCCCCAAGCCTGACCACCTAGGGAAAAGGCTCACCGTTCCCATGTGTG
GCTGATAAGGGCCAGGAGATTCCACAGTTCAGGTAGTTCCCCCGCCTCCCTGGCATT
TTGTGGTCACCATTAATCATTTCCTCTGTGTATTTAAGAGCTCTTTTGCCAGTGAGCCC
AGTACACAGAGAGAAAGGCTAAAGTTCTCTGGAGGAGCGGCCGCCCTATATAAGCA
GAGCTCGTTTAGTGAACCGTCAGATCGCCTGGAGA (SEQ ID NO: 22)
23. DNA sequence of pGM-CSF(-1 to -336).minCMV promoter 
TAAGTGTCTCCCACGCCCCACCCCAGCCATTCCAGGCCAGGAAGTCCAAAC
TGTGCCCCTCAGAGGGAGGGGGCAGCCTCAGGCCCATTCAGACTGCCCAGGGAGGG
CTGGAGAGCCCTCAGGAAGGCGGGTGGGTGGGCTGTCGGTTCTTGGAAAGGTTCAT
TAATGAAAACCCCCAAGCCTGACCACCTAGGGAAAAGGCTCACCGTTCCCATGTGTG
GCTGATAAGGGCCAGGAGATTCCACAGTTCAGGTAGTTCCCCCGCCTCCCTGGCATT
TTGTGGTCACCATTAATCATTTCCTCTGTGTATTTAAGAGCTCTTTTGCCAGTGAGCCG
CGGCCGCCCTATATAAGCAGAGCTCGTTTAGTGAACCGTCAGATCGCCTGGAGA (SEQ
ID NO: 23)
24. DNA sequence of pGM-CSF(-35 to -336).minCMV promoter 
TAAGTGTCTCCCACGCCCCACCCCAGCCATTCCAGGCCAGGAAGTCCAAAC
TGTGCCCCTCAGAGGGAGGGGGCAGCCTCAGGCCCATTCAGACTGCCCAGGGAGGG
CTGGAGAGCCCTCAGGAAGGCGGGTGGGTGGGCTGTCGGTTCTTGGAAAGGTTCAT
TAATGAAAACCCCCAAGCCTGACCACCTAGGGAAAAGGCTCACCGTTCCCATGTGTG
GCTGATAAGGGCCAGGAGATTCCACAGTTCAGGTAGTTCCCCCGCCTCCCTGGCATT
TTGTGGTCACCATTAATCATTTCCTGCGGCCGCCCTATATAAGCAGAGCTCGTTTAGT
GAACCGTCAGATCGCCTGGAGA (SEQ ID NO: 24)
25. DNA sequence of pGM-CSF(-1 to -217).minCMV promoter 
CAGGAAGGCGGGTGGGTGGGCTGTCGGTTCTTGGAAAGGTTCATTAATGAA
AACCCCCAAGCCTGACCACCTAGGGAAAAGGCTCACCGTTCCCATGTGTGGCTGATA
AGGGCCAGGAGATTCCACAGTTCAGGTAGTTCCCCCGCCTCCCTGGCATTTTGTGGT
 
CACCATTAATCATTTCCTCTGTGTATTTAAGAGCTCTTTTGCCAGTGAGCCGCGGCCG
CcCTATATAAGCAGAGCTCGTTTAGTGAACCGTCAGATCGCCTGGAGA (SEQ ID NO: 25)
26. DNA sequence of pGM-CSF(-35 to -217).minCMV promoter 
CAGGAAGGCGGGTGGGTGGGCTGTCGGTTCTTGGAAAGGTTCATTAATGAA
AACCCCCAAGCCTGACCACCTAGGGAAAAGGCTCACCGTTCCCATGTGTGGCTGATA
AGGGCCAGGAGATTCCACAGTTCAGGTAGTTCCCCCGCCTCCCTGGCATTTTGTGGT
CACCATTAATCATTTCCTGCGGCCGCcCTATATAAGCAGAGCTCGTTTAGTGAACCGTC
AGATCGCCTGGAGA (SEQ ID NO: 26)
27. DNA sequence of pGM-CSF(-35 to -179).minCMV promoter 
GTTCATTAATGAAAACCCCCAAGCCTGACCACCTAGGGAAAAGGCTCACCG
TTCCCATGTGTGGCTGATAAGGGCCAGGAGATTCCACAGTTCAGGTAGTTCCCCCGC
CTCCCTGGCATTTTGTGGTCACCATTAATCATTTCCTGCGGCCGCcCTATATAAGCAGA
GCTCGTTTAGTGAACCGTCAGATCGCCTGGAGA (SEQ ID NO: 27)
28. DNA sequence of pGM-CSF(-35 to -106).minCMV promoter 
GCCAGGAGATTCCACAGTTCAGGTAGTTCCCCCGCCTCCCTGGCATTTTGTG
GTCACCATTAATCATTTCCTGCGGCCGCcCTATATAAGCAGAGCTCGTTTAGTGAACC
GTCAGATCGCCTGGAGA (SEQ ID NO: 28)
29. DNA sequence of pGM-CSF(+36 to -217).minCMV promoter: 
CAGGAAGGCGGGTGGGTGGGCTGTCGGTTCTTGGAAAGGTTCATTAATGAA
AACCCCCAAGCCTGACCACCTAGGGAAAAGGCTCACCGTTCCCATGTGTGGCTGATA
AGGGCCAGGAGATTCCACAGTTCAGGTAGTTCCCCCGCCTCCCTGGCATTTTGTGGT
CACCATTAATCATTTCCTCTGTGTATTTAAGAGCTCTTTTGCCAGTGAGCCCAGTACA
CAGAGAGAAAGGCTAAAGTTCTCTGGAGGAGCGGCCGCcCTATATAAGCAGAGCTCG
TTTAGTGAACCGTCAGATCGCCTGGAGA (SEQ ID NO: 29)
30. DNA sequence of 6*NFAT.minIL2 promoter 
Ggaggaaaaactgtttcatacagaaggcgtcaattaggaggaaaaactgtttcatacagaaggcgtcaattaggaggaaaa
actgtttcatacagaaggcgtcaattggtcccatcgaattaggaggaaaaactgtttcatacagaaggcgtcaattaggaggaaaaactgttt
catacagaaggcgtcaattaggaggaaaaactgtttcatacagaaggcgtcaattggtcccgggacattttgacacccccataatatttttcc
agaattaacagtataaattgcatctcttgttcaagagttccctatcactctctttaatcactactcacagtaacctcaactcctga (SEQ ID
NO: 30)
31. DNA sequence of PA2 bidirectional PolyA
   AATAAAATATCTTTATTTTCATTACATCTGTGTGTTGGTTTTTTGTGTG (SEQ ID NO: 31)

All documents mentioned in the present application are cited herein for reference as if each document is individually cited for reference. Furthermore, it should be understood that after reading the foregoing teachings of the present application, those skilled in the art may make various alterations or modifications to the present application, and these equivalent forms also fall within the scope defined by the appended claims.

## Claims

1. A genetically modified promoter element, where the promoter element is a T cell activation induced promoter, and the promoter element has a fragment derived from a promoter selected from the group consisting of: GM-CSF promoter, IFNg promoter, IL2 promoter, and combinations thereof.

2. The promoter element according to claim 1, wherein the promoter element has a structure as shown in following formula (I):
Z0-Z1-Z2 (I)
In the formula, "-" represents a bond or nucleotide linker sequence;
Z0 is none, a fragment derived from a minimal promoter, or a fragment derived from other promoters;
Z1 is a fragment derived from a promoter selected from the group consisting of: GM-CSF promoter, IFNg promoter, IL2 promoter, and combinations thereof; and
Z2 is none, a fragment derived from a minimal promoter, or a fragment derived from other promoters.

3. The promoter element according to claim 2, wherein
(1) Z1 is a nucleic acid region from position p1 to q1 derived from a human GM-CSF promoter, wherein p1 is any integer from -800 to -100, and preferably from -400 to -150, and
q1 is any integer from -50 to 50, preferably from -40 to +40, and more preferably from -36 to +36; or
(2) Z1 is a nucleic acid region from position p2 to q2 derived from a human IL-2 promoter, wherein p2 is any integer from -500 to -200, and preferably from -400 to -300; and
q2 is any integer from 0 to +100, preferably from +30 to +70, and more preferably from +40 to +60; or
(3) Z1 is a nucleic acid region from position p3 to q3 derived from the IFNg promoter, wherein p3 is any integer from -800 to -100, and preferably from -600 to -400; and
q3 is any integer from +20 to +120, preferably from +40 to +80, and more preferably from +50 to +70.

4. The promoter element according to claim 2, wherein the minimal promoter is selected from the group consisting of: minimal CMV promoter, minimal IL2 promoter, synthetic minimal promoter, and combinations thereof.

5. The promoter element according to claim 1, wherein a nucleotide sequence of the promoter element is:
(a) a nucleotide sequence as shown in any one of SEQ ID NOs: 11-20 and 22-29;
(b) a nucleotide sequence having ≥90% (preferably ≥95%, more preferably ≥98%)homology to the sequence as shown in any one of SEQ ID NOs: 11-20 and 22-29, and having or substantially having a function of a polynucleotide as shown in any one of SEQ ID NOs: 11-20 and 22-29; or
(c) a polynucleotide having one or more, preferably 1-30, and more preferably 1-6 nucleotides truncated at a 5' end and/or 3' end of the polynucleotide as shown in any one of SEQ ID NOs: 11-20 and 22-29, and having or substantially having the function of the polynucleotide as shown in any one of SEQ ID NOs: 11-20 and 22-29.

6. An expression cassette, wherein the expression cassette has the promoter element according to claim 1, and a target gene ORF sequence operatively linked to the promoter element.

7. An expression cassette for co-expressing a chimeric antigen receptor (CAR) and an immunomodulatory factor, wherein the expression cassette has the promoter element according to claim 1, and the promoter element regulates expression of the immunomodulatory factor.

8. The expression cassette for co-expressing a CAR and an immunomodulatory factor according to claim 7, wherein the expression cassette has a structure as shown in following formula II from a 5' end to a 3' end:
CAR-PA2-IF-Prom (II)
in the formula,
each "-" independently represents a bond or a linker nucleic acid sequence;
CAR is a coding sequence of the chimeric antigen receptor;
PA2 is a bidirectional polyA sequence;
IF is a coding sequence of an immune regulatory factor in an inverse orientation; and
Prom is the promoter element according to claim 1 in an inverse orientation.

9. A vector, wherein the vector contains the expression cassette according to claim 6, or the expression cassette for co-expressing a CAR and an immunomodulatory factor according to claim 7.

10. An engineered immune cell, wherein the immune cell contains the vector according to claim 9, or a chromosome thereof is integrated with the expression cassette according to claim 6 or the expression cassette for co-expressing a CAR and an immunomodulatory factor according to claim 7.

11. The engineered immune cell according to claim 10, wherein the immune cell is T cell.

12. A method for preparing an engineered immune cell, wherein the method comprises following steps of: transducing the expression cassette according to claim 6, or the expression cassette for co-expressing a CAR and an immunomodulatory factor according to claim 7, or the vector according to claim 9 into an immune cell, to thereby obtain the engineered immune cell.

13. A pharmaceutical composition, wherein the pharmaceutical composition comprises the vector according to claim 9 and/or the engineered immune cell according to claim 10, and a pharmaceutically acceptable carrier, diluent or excipient.

14. The vector according to claim 9 or the engineered immune cell according to claim 10, for use in preparation of a medicament or a formulation for preventing and/treating a disease.

15. The vector or engineered immune cell used according to claim 14, wherein the disease is a tumor with high expression of B7-H3.

16. A method of treating a disease, comprising administering to a subject in need of treatment an effective amount of the engineered immune cell according to claim 10 or the pharmaceutical composition according to claim 13.
